# EUROPEAN PATENT APPLICATION

(11) **EP 2 687 193 A2**
(43) Date of publication of application: **22.01.2014**
(21) Application number: 12760161.5
(22) Date of filing: 09.02.2012
(51) Int. Cl.: A61F 9/06, A61F 9/04

(54) **ANTIGLARE APPARATUS HAVING A SELF-DIAGNOSIS MODE**

(30) Priority: 18.03.2011 KR 20110024478
(71) Applicant: Otos Wing.co., Ltd, Seoul 153-023 (KR)
(72) Inventor: HUH, Moon Young, Seoul 158-050 (KR)
(74) Representative: Viering, Jentschura & Partner
(86) International application number: PCT/KR2012/000961
(87) International publication number: WO 2012/128475

(57) **Abstract**

Disclosed herein is an anti-glare device having a self-diagnostic mode capable of preventing an operation trouble by previously self-diagnosing a work environment and an operating condition, of enhancing work convenience by displaying a remaining battery charge, and of immediately executing a self-diagnostic mode by one-touch input. The anti-glare device includes an optical detection unit, an electromagnetic wave sensor unit, an electromagnetic wave detection unit, a control unit, a light transmission control unit, and a self-diagnostic command input unit for recognizing signals to check whether or not the device is operating normally, a shade level, a sensitivity level, a delay level, and a remaining battery charge by user's direct selection, operation, or adjustment, and inputting the recognized signals to the control unit.

## Description

### [Technical Field]

The present invention relates to an anti-glare device, and more particularly to an anti-glare device having a self-diagnostic mode capable of preventing an operation trouble by previously self-diagnosing a work environment and an operating condition, of enhancing work convenience by displaying a remaining battery charge, and of immediately executing a self-diagnostic mode by one-touch input.

### [Background Art]

In general, when welding, cutting, or grinding is performed, a worker wears a welding mask for blocking strong light and performs a work, in order to protect the worker's eyes from various types of harmful materials. Accordingly, various convenient and safe electronic welding masks have been developed and utilized.

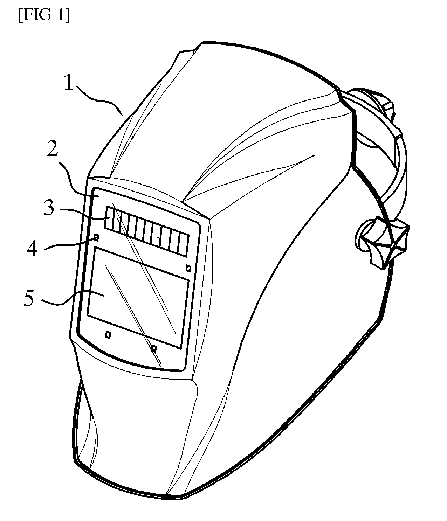

FIG. 1 is a perspective view of a conventional protective mask including an anti-glare device.

As shown in FIG. 1, the protective mask 1 including the anti-glare device 2 formed on a front surface thereof reduces illumination intensity of light irradiated to the worker's eyes through an anti-glare plate 5 which is an LCD (liquid crystal display) included in the anti-glare device 2.

That is, a photo-sensor unit 4 such as a photodiode included in a front surface of the anti-glare device 2 senses light generated by a welding and cutting torch. Thus, a control circuit integrated into the anti-glare device 2 controls the anti-glare plate 5 to be darkened such that illumination intensity of light passing through the anti-glare plate is reduced. Consequently, the worker's eyes are protected by the protective mask 1.

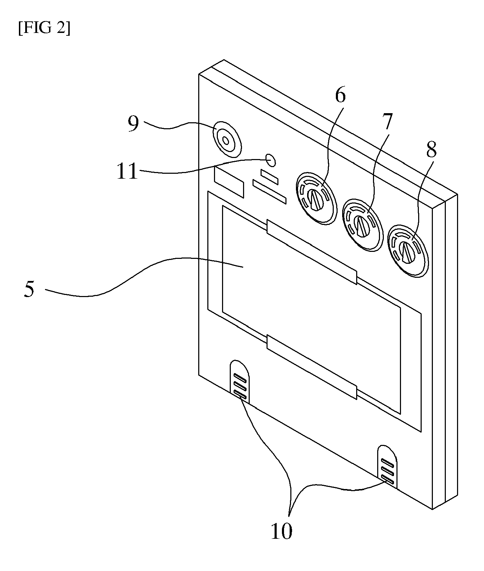

FIG. 2 is a view illustrating a user interface for adjusting shade, light detection sensitivity, and time delay of the conventional anti-glare device.

Referring to FIG. 2, the user interface of the conventional anti-glare device 2 includes a shade adjustment unit 6, a light detection sensitivity adjustment unit 7, and a time delay adjustment unit 8.

The shade adjustment unit 6 adjusts a shade value of the anti-glare plate 5. The shade value refers to a darkness degree of the anti-glare plate 5. When the shade value is adjusted by the shade adjustment unit 6, light transmissivity of the anti-glare plate 5 is adjusted.

The light detection sensitivity adjustment unit 7 adjusts light detection sensitivity of the anti-glare device 2. The light detection sensitivity refers to a value indicating a degree to which the control circuit of the anti-glare device 2 responds to an output signal of the photo-sensor unit 4. If the light detection sensitivity level is high, the control circuit may respond to the output signal with low illumination intensity.

The time delay adjustment unit 8 adjusts time delay of the anti-glare device 2. In a case in which the time delay level is low, the control circuit of the anti-glare device 2 rapidly switches the anti-glare plate 5 from a dark state to a bright state when the photo-sensor unit 4 senses that welding is finished. In contrast, when the time delay level is high, it takes substantial time to switch the anti-glare plate 5 from the dark state to the bright state.

Typically, in industries associated with the anti-glare device, the shade value level is 5 to 13, the light detection sensitivity level is 0 to 10, and the time delay level is 0 to 10. In addition, the user interface of the conventional anti-glare device 2 includes a power switch 9 for turning power on/off, a battery 10 for supplying power, and a low voltage indicator 11 for indicating a low voltage state of the device.

Meanwhile, the present applicant developed various anti-glare devices. For example, Korean Patent Application No. 10-2004-0052291, filed on July 6, 2004 discloses an electromagnetic wave detection anti-glare device for detecting electromagnetic waves generated together with light of high illumination intensity by a welding or cutting torch and protecting the worker's eyes. In addition, Korean Patent Application No. 10-2005-0127844 filed on December 22, 2005 discloses an anti-glare device using voice recognition control, which enables convenient control of various functions without any need for a worker to use their hands during welding and reports a control and operation state thereof vocally by performing a welding light blocking function using voice recognition technology, and a method of controlling the same.

However, in the above-mentioned conventional anti-glare device, there are problems in that various operation troubles occur since a work environment and an operating condition may not be previously self-diagnosed and power is suddenly turned off during working since a remaining battery charge is not displayed. In addition, there is a problem in that input work for recognizing an operation state one by one is complicated.

### [Disclosure]

### [Technical Problem]

Therefore, the present invention has been made in view of the above problems, and it is an object of the present invention to provide an anti-glare device having a self-diagnostic mode capable of preventing an operation trouble by previously self-diagnosing a work environment and an operating condition, of enhancing work convenience by displaying a remaining battery charge, and of immediately executing a self-diagnostic mode by one-touch input.

### [Technical Solution]

In accordance with an aspect of the present invention, the above and other objects can be accomplished by the provision of an anti-glare device having a self-diagnostic mode including an optical detection unit for detecting light generated by a welding or cutting torch, an electromagnetic wave sensor unit for sensing electromagnetic waves generated by the welding or cutting torch, an electromagnetic wave detection unit for comparing a resonated signal received through the electromagnetic wave sensor unit with a variably set reference value, when an electromagnetic wave detection unit operation signal is applied, a control unit for applying the electromagnetic wave detection unit operation signal to the electromagnetic wave detection unit and monitoring variation in an electromagnetic wave signal received via the electromagnetic wave detection unit, when optical detection is initiated by the optical detection unit, a light transmission control unit for controlling variation in light transmissivity of an anti-glare plate depending upon an output signal of the control unit, and a self-diagnostic command input unit for recognizing signals to check whether or not the device is operating normally, a shade level, a sensitivity level, a delay level, and a remaining battery charge by user's direct selection, operation, or adjustment, and inputting the recognized signals to the control unit.

The control unit may determine whether or not the device is operating normally, the shade level, the sensitivity level, the delay level, and the remaining battery charge depending upon commands received via the self-diagnostic command input unit.

The anti-glare device having a self-diagnostic mode may further include an operation status display unit for displaying whether or not the device is operating normally, a shade level display unit for displaying the shade level, a sensitivity display unit for displaying the sensitivity level, a delay display unit for displaying the delay level, and a remaining battery charge display unit for displaying the remaining battery charge.

### [Advantageous Effects]

In accordance with an anti-glare device having a self-diagnostic mode according to the present invention, it may be possible to prevent an operation trouble by previously self-diagnosing a work environment and an operating condition, to enhance work convenience by displaying a remaining battery charge, and to immediately execute a self-diagnostic mode by one-touch input.

### [Description of Drawings]

The above and other objects, features and other advantages of the present invention will be more clearly understood from the following detailed description taken in conjunction with the accompanying drawings, in which:
FIG. 1 is a perspective view of a conventional protective mask including an anti-glare device;
FIG. 2 is a view illustrating a user interface for adjusting shade, light detection sensitivity, and time delay of the conventional anti-glare device;
FIG. 3 is a block diagram illustrating an anti-glare device having a self-diagnostic mode according to the present invention; and
FIG. 4 is a front view illustrating the anti-glare device having a self-diagnostic mode according to the present invention.

### [Best Mode]

Reference will now be made in detail to the preferred embodiment of the present invention with reference to the attached drawings.

The terms or words used in the specification and claims of the present invention are not to be construed as limited to ordinary or dictionary meanings, but should be construed as meanings and concepts conforming to the technical spirit of the present invention on the basis of the principle that the inventors can define the concept of the terms properly to explain their invention with the best method. Accordingly, it is to be understood that the detailed description which will be disclosed along with the accompanying drawings is intended to describe an exemplary embodiment of the present invention, and is not intended to describe a unique embodiment which the present invention can be carried out. Therefore, various equivalents and modifications are possible within the scope of the present invention at the time of filing.

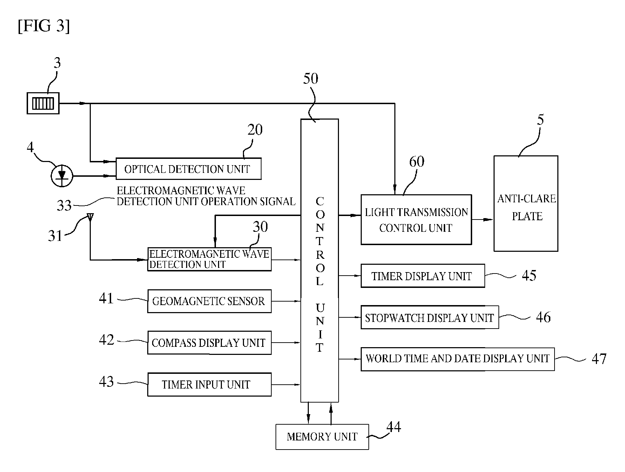

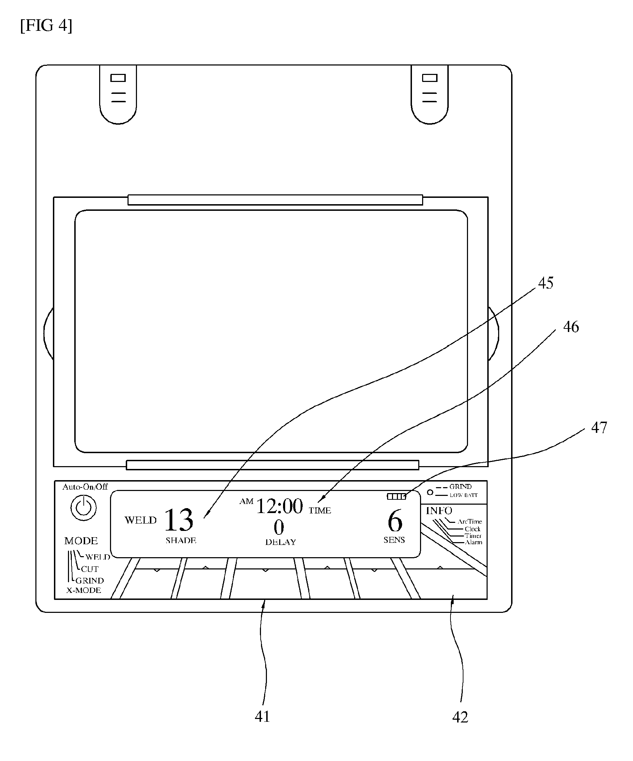

FIG. 3 is a block diagram illustrating an anti-glare device having a self-diagnostic mode according to the present invention. FIG. 4 is a front view illustrating the anti-glare device having a self-diagnostic mode according to the present invention.

As shown in the drawings, the anti-glare device according to the present invention includes an optical detection unit 20, an electromagnetic wave detection unit 30, an electromagnetic wave sensor unit 31, a self-diagnostic command input unit 41, an operation status display unit 42, a shade level display unit 43, a memory unit 44, a sensitivity display unit 45, a delay display unit 46, a remaining battery charge display unit 47, a control unit 50, and a light transmission control unit 60.

The optical detection unit 20 detects light generated by a welding or cutting torch and includes a filter and an amplifier. The optical detection unit 20 compares a signal received via a photo-sensor unit 4 with output of a solar battery 3 and detects variation in light quantity.

The electromagnetic wave detection unit 30 detects electromagnetic waves generated by the welding or cutting torch. The electromagnetic wave detection unit 30 compares a resonated and filtered signal received through the electromagnetic wave sensor unit 31, which senses the electromagnetic waves generated by the welding or cutting torch of a worker, with a preset value so as to detect electromagnetic waves having a specific bandwidth.

When optical detection is initiated by the optical detection unit 20, the control unit 50 applies an electromagnetic wave detection unit operation signal 33 to the electromagnetic wave detection unit 30 and monitors variation in an electromagnetic wave signal received via the electromagnetic wave detection unit.

The light transmission control unit 60 controls variation in light transmissivity of an anti-glare plate 5 depending upon an output signal of the control unit 50.

The present invention includes the self-diagnostic command input unit 41 in order to implement a self-diagnostic function. The self-diagnostic command input unit 41 allows a user to input a variety of commands required for selfdiagnosis.

In detail, the self-diagnostic command input unit 41 recognizes signals to check whether or not the device is operating normally, a shade level, a sensitivity level, a delay level, and a remaining battery charge by user's direct selection, operation, or adjustment, and inputs the recognized signals to the control unit 50.

Thus, the control unit 50 determines whether or not the device is operating normally, the shade level, the sensitivity level, the delay level, and the remaining battery charge depending upon the commands received via the self-diagnostic command input unit.

The memory unit 44 stores whether or not the device is operating normally, the shade level, the sensitivity level, the delay level, and the remaining battery charge, which are determined by the control unit 50.

In addition, the operation status display unit 42 displays whether or not the device is operating normally, the shade level display unit 43 displays the shade level, the sensitivity display unit 45 displays the sensitivity level, the delay display unit 46 displays the delay level, and the remaining battery charge display unit 47 displays the remaining battery charge.

Hereinafter, a preferred embodiment of the present invention having such configurations will be described in more detail.

First, the optical detection unit 20 detects welding light through the signal received via the photo-sensor unit 4, and more particularly detects light generated by the welding or cutting torch.

Subsequently, the control unit 50 sets a reference value for controlling detection sensitivity of the optical detection unit 20 according to an input value of the user, sets a light transmission density and operation a delay time of the anti-glare plate 5, and determines that the detected light is the welding light when the quantity of light detected by the optical detection unit 20 is equal to or greater than a preset reference value, thereby operating the light transmission control unit 60.

Accordingly, the light transmission control unit 60 operates the anti-glare plate 5 depending upon the light transmission density set by the control unit 50 so as to control light transmissivity to a predetermined value or less.

Meanwhile, the user inputs signals to check whether or not the device is operating normally, the shade level, the sensitivity level, the delay level, and the remaining battery charge to the control unit 50 by directly selecting, operating, or adjusting the self-diagnostic command input unit 41.

Thus, the control unit 50 determines whether or not the device is operating normally, the shade level, the sensitivity level, the delay level, and the remaining battery charge depending upon the commands received via the self-diagnostic command input unit.

In addition, the memory unit 44 stores whether or not the device is operating normally, the shade level, the sensitivity level, the delay level, and the remaining battery charge, which are determined by the control unit 50.

The determined resultant values may be displayed by the respective display units.

As an example, the operation status display unit 42 displays whether or not the device is operating normally, the shade level display unit 43 displays the shade level, the sensitivity display unit 45 displays the sensitivity level, the delay display unit 46 displays the delay level, and the remaining battery charge display unit 47 displays the remaining battery charge.

Thus, in accordance with the anti-glare device having a self-diagnostic mode according to the present invention, it may be possible to prevent an operation trouble by previously self-diagnosing a work environment and an operating condition, to enhance work convenience by displaying the remaining battery charge, and to immediately execute a self-diagnostic mode by one-touch input.

Various embodiments have been described in the best mode for carrying out the invention. Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An anti-glare device having a self-diagnostic mode, comprising:
an optical detection unit for detecting light generated by a welding or cutting torch;
an electromagnetic wave sensor unit for sensing electromagnetic waves generated by the welding or cutting torch;
an electromagnetic wave detection unit for comparing a resonated signal received through the electromagnetic wave sensor unit with a variably set reference value, when an electromagnetic wave detection unit operation signal is applied;
a control unit for applying the electromagnetic wave detection unit operation signal to the electromagnetic wave detection unit and monitoring variation in an electromagnetic wave signal received via the electromagnetic wave detection unit, when optical detection is initiated by the optical detection unit;
a light transmission control unit for controlling variation in light transmissivity of an anti-glare plate depending upon an output signal of the control unit; and
a self-diagnostic command input unit for recognizing signals to check whether or not the device is operating normally, a shade level, a sensitivity level, a delay level, and a remaining battery charge by user's direct selection, operation, or adjustment, and inputting the recognized signals to the control unit.

2. The anti-glare device having a self-diagnostic mode according to claim 1, wherein the control unit determines whether or not the device is operating normally, the shade level, the sensitivity level, the delay level, and the remaining battery charge depending upon commands received via the self-diagnostic command input unit.

3. The anti-glare device having a self-diagnostic mode according to claim 1, further comprising:
an operation status display unit for displaying whether or not the device is operating normally;
a shade level display unit for displaying the shade level;
a sensitivity display unit for displaying the sensitivity level;
a delay display unit for displaying the delay level; and
a remaining battery charge display unit for displaying the remaining battery charge.
